# EUROPEAN PATENT SPECIFICATION

(11) **EP 3 879 791 B1**
(45) Date of publication and mention of the grant of the patent: **29.05.2024**
(21) Application number: 20162153.9
(22) Date of filing: 10.03.2020
(51) Int. Cl.: H04W 4/38, H04W 4/70, A61G 13/00, H04W 88/16

(54) **SURGICAL TABLE DATA TRANSMISSION SYSTEM**
DATENÜBERTRAGUNGSSYSTEM FÜR EINEN OPERATIONSTISCH
SYSTÈME DE TRANSMISSION DE DONNÉES DE TABLE CHIRURGICALE

(43) Date of publication of application: 15.09.2021
(73) Proprietor: Baxter Medical Systems GmbH + Co. KG, 07318 Saalfeld (DE)
(72) Inventor: BERNOTSKY, Jason, Batesville, Indiana 47006 (US); PIERCE, Michael C., Batesville, Indiana 47006 (US); DOLLIVER, Phillip, Batesville, Indiana 47006 (US)
(74) Representative: Loustalan, Paul William

(56) References cited:
- WO-A1-2013/123119
- US-A1- 2007 010 719
- US-A1- 2018 350 464

## Description

The invention relates to a surgical table data transmission system, in particular, to a surgical table data transmission system comprising using a data network.

Currently, beyond a basic presence detection, the majority of surgical tables do not have any smart sensing or control features integrated with removable components or accessories. However, the lack of these capabilities limits the ability of the surgical tables to lock out certain unsafe movements.

Moreover, due to a lot of various combinations, a multitude of table components and accessories require a more complex control for the table which is accompanied by huge effort for wiring and configuration of controllers required.

WO2013123119 A1 discloses a patient support system. The electronic control system may include a gateway module, or similar type of module, that allows for communications with a healthcare computer system or network, such as a hospital Ethernet, or other facility computer network. The gateway module controller could be electrically coupled to electronic communication network so that is could send and receive information from any of the electronic controllers, modules, or other devices that communicate over network. Information related to, or generated by, any of load cells, force sensors, gesture sensors, and/or remote control can therefore be transmitted off of patient support apparatus to a healthcare network.

US 2007/010719 A1 discloses a method and system for remote diagnostic monitoring of a healthcare device, for example, a patient support system. The system includes a communication device configured to transmit sensor data received from a sensor system coupled to the patient support system and configured to generate sensor data relating to diagnostic information of the healthcare device. The communication device can also be configured to receive configuration data for configuring at least one of the healthcare device and the sensor system. The monitoring device is located geographically distant from the healthcare device, is coupled to the communication adapter by a datalink, for example, a telecommunication network, and is configured to receive the sensor data. The monitoring device is configured to determine, based on the received data, whether service or replacement of the healthcare device or a portion thereof is indicated.

US 2018/350464 A1 discloses patient care devices, including beds, cots, stretchers, recliners, chairs, thermal control systems, and IV poles, include a user interface for controlling an aspect of the patient care device, a port for communicating with an external device, and a control system. The control system determines when an external device is communicating with the port, what type of device the external device is, and if the patient care device includes software for allowing the user interface to act as a user interface of the external device. In some embodiments, the control system receives a device type identifier when an external device is coupled to the port, selects a software module for communicating with the coupled external device, and uses the selected software module for communicating with the coupled external device. Multiple external devices may be connected to the port and display data on a display of the patient care device.

The object underlying the invention is to remedy these disadvantageous and to provide a surgical table data transmission system, a component of a surgical table, an accessory of the surgical table, and a surgical table enabling a flexible data exchange between a data processing device and components of the surgical table.

The object is achieved by a surgical table data transmission system according to claim 1, a component or an accessory according to claim 5, and a surgical table according to claim 11.

According to an aspect of the invention, a surgical table data transmission system comprises a plurality of remote nodes, wherein each remote node it attached to a component or an accessory of a surgical table and a data aggregation node, wherein each of the plurality of remote nodes is configured to exchange data with other remote nodes and with the data aggregation node by means of wired or wireless communication, each of the plurality of remote nodes is configured to transmit identification data of the component or accessory to the data aggregation node, either indirectly via at least one other remote node, or directly with the data aggregation node, and the data aggregation node is configured to exchange the data with at least one of a controller of the surgical table and a hospital network gateway by a second wired or wireless communication.

The nodes are to be understood as network nodes hardware which can receive, create, store or send data along distributed network routes.

By the data transmission system, data, such as identifiers, e.g., stored identification data, received sensor data or inputs by an operator, can be transmitted from several components or accessories of the surgical table via an assigned remote node which can be attached to the component or accessory and the data aggregation node to the controller of the surgical table or to the hospital network gateway. On the other hand, control signals from the controller of the surgical table or information of the hospital network can be transmitted via the data aggregation node and the remote node to actors or displays on the components or accessories of the surgical table. Therefore, a transmission of data can be performed without the necessity of individual data lines which enables a lean structure of the data transfer and a flexibility in case of a change of the configuration of the surgical table.

The data can be transmitted wirelessly or in a wired manner. In case of the wired manner, the principle of plug and receptacle is used or contacts which automatically establish a contact when attaching the component or the accessory to the surgical table are provided. In this case, for example, a RS-485 or a CAN protocol is used.

In an advantageous implementation of the surgical table data transmission system, the system comprises a plurality of remote nodes, each remote node being configured to be able to exchange data with each other remote node (8) and with the data aggregation node. At least one first remote node directly exchanges data with the data aggregation node, and at least one second remote node indirectly exchanges data with the data aggregation node via the at least one first remote node.

In a further advantageous implementation of the surgical table data transmission system, the remote node is configured to transmit sensor data from the component or the accessory to the data aggregation node.

In yet a further advantageous implementation of the surgical table data transmission system, the data aggregation node and the remote node respectively comprise at least one of a transmitter and a receiver for radio communication between the data aggregation node and the remote node.

Due to this configuration, the wireless communication between the data aggregation node and the remote node is possible. Therefore, no wiring is necessary so that the effort for wiring can be saved and, also, there is no risk for damaging a cable. Therefore, safety of the operation of the surgical table is enhanced. As transmission protocols for the communication, for example, Zigbee, Near Field Communication (NFC), Bluetooth or WLAN can be used.

In yet a further advantageous implementation of the surgical table data transmission system, the data aggregation node comprises at least one of a transmitter and a receiver for radio communication with the controller of the surgical table or the hospital network gateway.

By this configuration, the wireless communication between the data aggregation node and the controller of the surgical table or the hospital network gateway is possible. Therefore, no wiring is necessary so that the effort for wiring can be saved and, also, there is no risk for damaging a cable. Therefore, safety of the operation of the surgical table is enhanced. As a transmission protocol for the communication, for example, WLAN can be used.

According to a further aspect of the invention, a component of a surgical table is provided, wherein the component comprises the remote node of the surgical table data transmission system.

By providing the component of the surgical table with the remote node, specific information concerning the component can be provided by the remote node and, as the case may be, functions of the component, such as motions by a controlled drive, can be performed by being controlled via the remote node.

In an advantageous implementation of the component, the component comprises a memory device configured to store the identification data of the component.

By the identification data of the component stored in the memory device thereof, the controller of the surgical table can recognize the type of the component and can adjust control signals in order to, e.g., avoid collisions with other components of the surgical table or with the floor. Also, an automatic recognition of the component when being attached to the surgical table is possible.

In a further advantageous implementation of the component, the component comprises a sensor configured to transmit sensor data to the remote node.

Due to the component comprising the sensor, for example, monitoring a patient lying on the component of the surgical table is possible. Furthermore, monitoring the component for avoiding damage, e.g., by overload or collision, is possible. By the transmission of the sensor signals via the remote node and the data aggregation node, no further signal path to the controller of the surgical table is necessary so that additional efforts can be saved.

In a further advantageous implementation of the component, the sensor is at least one of a pressure mapping sensor, a temperature sensor, a patient weight sensor, an angle measuring sensor, and a collision detection sensor.

By deploying the pressure mapping sensor, the temperature sensor and/or the patient weight sensor, the patient lying on the component of the surgical table can be monitored in order to, e.g., avoid decubitus or hypothermia during a surgical intervention. By the use of the angle measuring sensor and/or the collision detection sensor, the patient and the surgical staff can be kept safe since inadmissible states of the surgical table can be determined.

In a yet further advantageous implementation of the component, the component comprises a battery configured to supply energy to the remote node.

When providing the component with a battery, no line for supplying energy to the remote node or, as the case may be, the sensor is necessary. Therefore, for this purpose, no wiring is necessary so that this effort can be saved and, also, there is no risk for damaging a cable which enhances safety of the operation of the surgical table.

In a further advantageous implementation of the component, the component is at least one of a head plate, a leg plate, a seat plate and a back plate.

According to a further aspect of the invention, an accessory of a surgical table is provided, wherein the accessory comprises the remote node of the surgical table data transmission system.

By providing the accessory of the surgical table with the remote node, an automatic recognition of the accessory when being attached to the surgical table is possible. Further, specific information of the accessory can be provided by the remote node and, as the case may be, functions of the accessory, such as motions executed by a controlled drive, can performed by being controlled via the remote node.

In an advantageous implementation of the accessory, the accessory comprises a memory device configured to store the identification data of the accessory.

By the identification data of the accessory stored in the memory device thereof, for example, the controller of the surgical table can recognize the type of the accessory and can adjust control signals in order to, e.g., avoid collisions with components of the surgical table or with the floor.

In a further advantageous implementation of the accessory, the accessory comprises a sensor configured to transmit sensor data to the remote node.

Due to the accessory comprising the sensor, for example, monitoring a patient being in contact with the accessory of the surgical table is possible. Furthermore, monitoring the accessory for avoiding damage, e.g., by overload or collision, is possible. By the transmission of the sensor signals to the remote node, no further signal path to the controller of the surgical table is necessary.

In a further advantageous implementation of the accessory, the accessory comprises a battery configured to supply energy to the remote node.

When providing the accessory with a battery, no line for supplying energy to the remote node or the sensor is necessary. Therefore, no wiring therefore is necessary so that this effort can be saved and, also, there is no risk for damaging a cable which enhances safety of the operation of the surgical table.

In a further advantageous implementation of the accessory, the accessory is at least one of a pad with pressure sensing and an electrically adjustable support device.

According to a further aspect of the invention, a surgical table comprises a surgical table data transmission system, at least one of a component and an accessory, and a controller of the surgical table, wherein the controller is configured to control functions of the surgical table. The data aggregation node is configured to exchange the system data with the controller.

In the surgical table equipped in this manner, the data can be transmitted, if provided, from several components or accessories to the controller of the surgical table via a remote node assigned to the component or the accessory and which can be attached to the component or the accessory and the data aggregation node. On the other hand, control signals or information of the controller can be transmitted via the data aggregation node and the remote node to actors or displays on the components or accessories of the surgical table. Therefore, the transmission of data can be performed without the necessity of individual data lines which enables a lean structure of the data transfer and a flexibility in case of a change of the configuration of the surgical table.

According to an advantageous implementation of the surgical table, the data aggregation node is integrated in the controller of the surgical table.

By the integration of the data aggregation node in the controller of the surgical table, no further device, as the case may be, comprising a separate casing, is necessary in order to avoid additional efforts.

According to another advantageous implementation of the surgical table, the data aggregation node is a separate device connected to the controller of the surgical table.

When providing the data aggregation node as a separate device, for example as an upgrade device or dongle, an upgrade of the functionality of the surgical table is possible without big additional efforts.

Below, the invention is elucidated in detail by means of embodiments referring to the attached drawings.

In particular,
- Fig. 1: shows a surgical table according to the invention;
- Fig. 2: shows a surgical table component identification and sensing system connection diagram; and
- Fig. 3: shows a remote identification/sensing node basic electrical diagram.

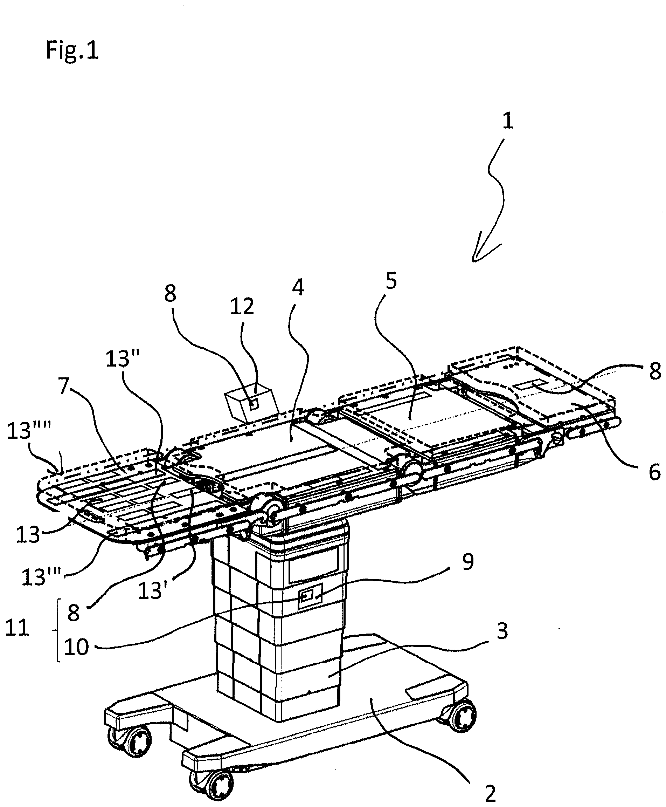

**Fig. 1** shows a surgical table 1 according to the invention. The surgical table 1 comprises a base 2, a column 3, a seat plate 4 and a back plate 5. Nevertheless, the surgical table 1 can alternatively have another structure.

Further, the surgical table 1 comprises a head plate 6 and a leg plate 7 as components which are detachable. In alternative embodiments, merely some of these components are provided, not all of these components are detachable, no detachable component is provided or other or additional components are provided.

The detachable components 6, 7 are respectively provided with a remote identification/sensing node as a remote node 8. In alternative embodiments, not all of the detachable components 6, 7 are provided with a remote node 8 or components of the surgical table 1 which are not detachable, such as the seat plate 4 or the back plate 5, are also provided with the remote node 8.

In the shown embodiment, the leg plate 7 is provided with a pressure mapping sensor 13 configured to detect a pressure distribution on a surface of the leg plate 7. Further, the leg plate 7 is provided with a temperature sensor 13' for measuring a body temperature of a patient lying on the tabletop, a patient weight sensor 13", an angle measurement sensor 13‴ for measuring an inclination of the component 7, and a collision detection sensor 13ʺʺ detecting a collision between the component 7 and another component of the surgical table 1 or the environments of the surgical table 1. In alternative embodiments, the detachable components 6, 7 comprise other sensors, all of the components 4, 5, 6, 7 are respectively provided with at least one sensor 13, 13', 13", 13'", 13"", or none of the components 4, 5, 6, 7 is provided with a sensor.

The surgical table 1 further comprises an accessory 12 which is illustrated in a principal manner. The accessory 12 is a pad with pressure sensing. Alternatively, the accessory 12 can be an electrically adjustable support device. The accessory 12 is also provided with one of the remote nodes 8. In alternative embodiments, several accessories 12 having a remote node 8 are provided, no accessory 12 is provided or not all of the accessories 12 are provided with the remote node 8.

The surgical table 1 comprises a controller 9 which is configured to control functions of the surgical table 1. In particular, the controller 9 controls motions of the surgical table 1, such as a height adjustment of a tabletop comprising the seat plate 4, the back plate 5, the head plate 6 and the leg plate 7. The controller 9 further controls tilting of the tabletop around its longitudinal axis and tilting of the tabletop around an axis perpendicular to the longitudinal axis into a Trendelenburg or Anti-Trendelenburg position.

The controller 9 comprises an integrated central control node as data aggregation node 10. The data aggregation node 10 exchanges data with the controller 9. Alternatively, the data aggregation node 10 is a separate device connected to the controller 9 of the surgical table. Such a separate device is, for example, an upgrade device or a dongle. Also, alternatively, the separate data aggregation node 10 can be provided in a wall control unit, a surgical light, etc..

In an alternative embodiment in which the non-detachable components 4, 5 are provided with sensors, these sensors are either directly connected to the controller 9 or connected to the controller 9 also via the remote node 8 and the data aggregation node 10.

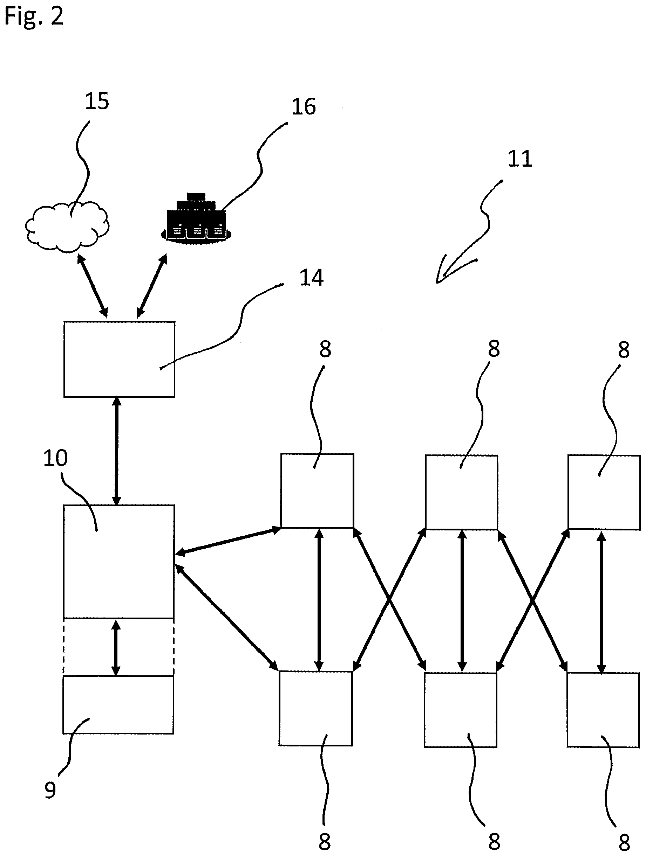

**Fig. 2** shows a surgical table component identification and sensing system connection diagram.

The surgical table component identification and sensing system connection diagram shows a surgical table data transmission system 11 comprising the data aggregation node 10 and the remote nodes 8. As mentioned above, the data aggregation node 10 is integrated in the controller 9 of the surgical table 1 and the remote nodes 8 are attachable to the components 6, 7.

The data aggregation node 10 and the remote nodes 8 exchange data by a wireless communication. The remote nodes 8 also exchange data among each other. Thereto, the data aggregation node 10 and the remote nodes 8 respectively comprise a transmitter and/or a receiver for radio communication. In this embodiment, the data exchange is performed via WLAN. Alternatively, "Zigbee", "Near field Communication" or "Bluetooth" can be used for the wireless communication or a CAN or a RS-485 protocol can be used for a wired communication.

The data aggregation node 10, on the one hand, exchanges system data with the controller 9 of the surgical table 1 and/or, on the other hand, with a hospital network gate way 14. The hospital network gateway 14 is connected to a hospital cloud 15 and/or a hospital data network 16. The data are either directly forwarded from one of the remote nodes 8 or processed by the data aggregation node 10 or the controller 9 of the surgical table 1. The data exchange between the data aggregation node 10 and the hospital network gateway 14 is performed by a wired communication via the RS-485 protocol. Alternatively, another protocol, such as CAN, or a wireless communication, e.g., via WLAN, is used. Then, the data aggregation node 10 comprises at least one of a transmitter and a receiver for radio communication to the controller 9 of the surgical table 1 or the hospital network gateway 14.

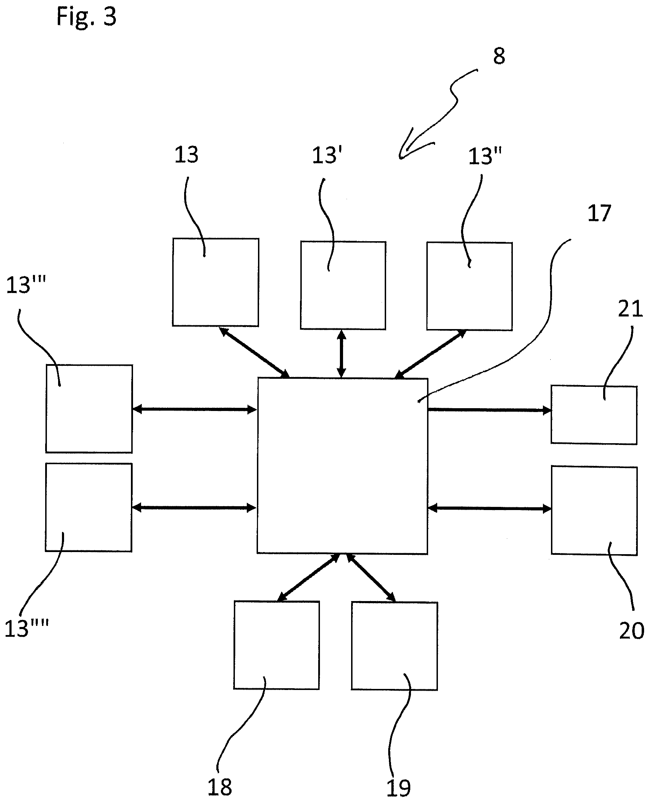

**Fig. 3** shows a remote identification/sensing node basic electrical diagram of the remote node 8.

Fig. 3 shows that a microcontroller 17 is connected to the pressure mapping sensor 13, the temperature sensor 13', the patient weight sensor 13", the angle measurement sensor 13"', and the collision detection sensor 13"". The sensors 13, 13', 13", 13'", 13ʺʺ are provided in/on the components 6, 7 or the accessory 12. The sensors 13, 13', 13", 13'", 13ʺʺ transmit sensor data to the remote node 8. As mentioned above, alternatively, not all of these sensors are provided, or some of these sensors or other sensors are provided in/on the components 6, 7 or the accessory 12.

The microcontroller 17 is further connected to a battery 18 configured to supply energy to the remote node 8. The battery 18 is provided in/on the component 6, 7 and/or the accessory 12 for supplying the remote node 8 and, as the case may be, the sensors with energy. Alternatively, no battery 18 is provided and the remote node 8 is, for example, supplied with energy by wiring.

Furthermore, the microcontroller 17 is connected to a wireless interface 19. The interface 19 serves for the data exchange with another remote nodes 8 or with the data aggregation node 10 as already mentioned above.

Moreover, the microcontroller 17 is connected to a memory device 20 which stores the identification data of the component 6, 7 and/or of the accessory 12. The component 6, 7 and/or the accessory 12 comprises the memory device 20. Alternatively, the identification data are not provided or stored in another manner, e.g., by a specific shape of a mechanical interface.

The microcontroller 17 is further connected to a simple local indicator 21 which serves as a user interface. Alternatively, no such simple local indicator 21 is provided on the component 5, 6 and the accessory 12.

In use, when starting up the controller 9 of the surgical table 1 or when attaching one of the components 6, 7 or the accessory 12 to the surgical table 1, the remote node 8 transmits data for a component or accessory type identification to the data aggregation node 10. Alternatively, this data can also be transmitted several times, e.g., cyclically. By this data, a configuration of the surgical table 1 can be determined and correct accessories 12 and components 6, 7 can be planned to be in place by hospital staff for a planned surgical procedure.

Furthermore, the remote node 8 transmits senor data of the sensors 13, 13', 13", 13"', 13"" to the data aggregation node 10. The sensor data are transmitted cyclically. This sensor data can be used to keep patient and surgical staff safe. Alternatively, the sensor data are not transmitted cyclically but only upon change of the sensor data and if, for example, no sensors are provided, no sensor data are transmitted.

The data is transmitted to the controller 9 of the surgical table 1 for appropriately controlling the surgical table 1. Furthermore, alternatively or additionally, the data is transmitted to the hospital network gateway 14 where it is forwarded to hospital staff for planning purposes.

While the invention has been illustrated and described in detail in the drawing and the foregoing description, such illustration and description are to be considered illustrative or exemplary and not restrictive.

In the claims, the word "comprising" does not exclude other elements or steps.

## Claims

1. A surgical table data transmission system (11) comprising
a plurality of remote nodes (8), wherein each remote node (8) is attached to a component (7) or an accessory (12), of a surgical table (1), and
a data aggregation node (10), wherein
each of the plurality of remote nodes (8) is configured to exchange data with other remote nodes (8) and with the data aggregation node (10) by a first wired or wireless communication,
each of the plurality of remote nodes (8) is configured to transmit identification data of the component (7) or accessory (12) to the data aggregation node (10), either indirectly via at least one other remote node, or directly with the data aggregation node (10), and
the data aggregation node (10) is configured to be able to exchange the data with at least one of a controller (9) of the surgical table (1) and a hospital network gateway (14) by a second wired or wireless communication.

2. The surgical table data transmission system (11) of claim 1, wherein the remote node (8) is further configured to transmit sensor data from the component (7) or the accessory (12) to the data aggregation node (10), either indirectly via at least one other remote node, or directly with the data aggregation node (10).

3. The surgical table data transmission system (11) of any preceding claim, wherein
the data aggregation node (10) and the remote node (8) respectively comprise at least one of a transmitter and a receiver for radio communication between the data aggregation node (10) and the remote node (8).

4. The surgical table data transmission system (11) of any preceding claim, wherein
the data aggregation node (10) comprises at least one of a transmitter and a receiver for radio communication with the controller (9) of the surgical table or the hospital network gateway (14).

5. A component (6, 7) of a surgical table (1), wherein the component (6, 7) comprises a remote node (8) of the surgical table data transmission system (1) of anyone of claims 1 to 4.

6. The component (6, 7) of claim 5, wherein
the component (6, 7) comprises a memory device (20) configured to store the identification data of the component (6, 7).

7. The component (6, 7) of claim 5 or 6, wherein
the component (6, 7) comprises a sensor (13 - 13"") configured to transmit sensor data to the remote node (8).

8. The component (6, 7) of claim 7, wherein
the sensor (13 - 13"") is at least one of a pressure mapping sensor (13), a temperature sensor (13'), a patient weight sensor (13"), an angle measuring sensor (13‴), and a collision detection sensor (13ʺʺ).

9. The component (6, 7) of anyone of claims 5 to 8, wherein
the component (6, 7) comprises a battery (18) configured to supply energy to the remote node(8).

10. The component (6, 7) of anyone of claims 5 to 9, wherein
the component (6, 7) is at least one of a head plate (6), a leg plate (7), a seat plate (4) and a back plate (5).

11. A surgical table (1) comprising
a surgical table data transmission system (11) of anyone of claims 1 to 4,
at least one of a component (6, 7) of anyone of claims 5 to 10,
a controller (9) of the surgical table (1), the controller (9) being configured to control functions of the surgical table (1), wherein
the data aggregation node (10) of the surgical table data transmission system is configured to exchange the data with the controller (9).

12. The surgical table (1) of claim 11, wherein
the data aggregation node (10) is a separate device connected to the controller (9) of the surgical table (1).

## Patentansprüche

1. Operationstisch-Datenübertragungssystem (11), das Folgendes umfasst:
mehrere entfernte Knotenpunkte (8), wobei jeder entfernte Knotenpunkt (8) an einer Komponente (7) oder einem Zubehörteil (12) eines Operationstischs (1) angebracht ist, und
einen Datenaggregationsknoten (10), wobei
jeder der mehreren entfernten Knotenpunkte (8) zum Austauschen von Daten mit anderen entfernten Knotenpunkten (8) und mit dem Datenaggregationsknoten (10) über eine erste verdrahtete oder drahtlose Kommunikation konfiguriert ist,
jeder der mehreren entfernten Knotenpunkte (8) zum Übertragen von Identifikationsdaten der Komponente (7) oder des Zubehörteils (12) zum Datenaggregationsknoten (10) entweder indirekt über mindestens einen anderen entfernten Knotenpunkt oder direkt mit dem Datenaggregationsknoten (10) konfiguriert ist, und
der Datenaggregationsknoten (10) so konfiguriert ist, dass er die Daten über eine zweite drahtgebundene oder drahtlose Kommunikation mit einer Steuerung (9) des Operationstischs (1) und/oder einem Krankenhausnetzwerk-Gateway (14) austauschen kann.

2. Operationstisch-Datenübertragungssystem (11) nach Anspruch 1, wobei der entfernte Knotenpunkt (8) ferner zum Übertragen von Sensordaten von der Komponente (7) oder dem Zubehörteil (12) zum Datenaggregationsknoten (10) entweder indirekt über mindestens einen anderen entfernten Knotenpunkt oder direkt mit dem Datenaggregationsknoten (10) konfiguriert ist.

3. Operationstisch-Datenübertragungssystem (11) nach einem vorherigen Anspruch, wobei
der Datenaggregationsknoten (10) und der entfernte Knotenpunkt (8) jeweils einen Sender und/oder einen Empfänger für Funkkommunikation zwischen dem Datenaggregationsknoten (10) und dem entfernten Knotenpunkt (8) umfassen.

4. Operationstisch-Datenübertragungssystem (11) nach einem vorherigen Anspruch, wobei
der Datenaggregationsknoten (10) einen Sender und/oder einen Empfänger für Funkkommunikation mit der Steuerung (9) des Operationstischs oder dem Krankenhausnetzwerk-Gateway (14) umfasst.

5. Komponente (6, 7) eines Operationstischs (1), wobei die Komponente (6, 7) einen entfernten Knotenpunkt (8) des Operationstisch-Datenübertragungssystems (1) nach einem der Ansprüche 1 bis 4 umfasst.

6. Komponente (6, 7) nach Anspruch 5, wobei
die Komponente (6, 7) ein Speichergerät (20) umfasst, das zum Speichern der Identifikationsdaten der Komponente (6, 7) konfiguriert ist.

7. Komponente (6, 7) nach Anspruch 5 oder 6, wobei
die Komponente (6, 7) einen Sensor (13 - 13"") umfasst, der zum Übertragen von Sensordaten zu dem entfernten Knotenpunkt (8) konfiguriert ist.

8. Komponente (6, 7) nach Anspruch 7, wobei
der Sensor (13 - 13"") mindestens einer von einem Druckabbildungssensor (13), einem Temperatursensor (13'), einem Patientengewichtssensor (13"), einem Winkelmesssensor (13‴) und einem Kollisionserkennungssensor (13"") ist.

9. Komponente (6, 7) nach einem der Ansprüche 5 bis 8, wobei
die Komponente (6, 7) eine Batterie (18) umfasst, die zum Versorgen des entfernten Knotenpunkts (8) mit Energie konfiguriert ist.

10. Komponente (6, 7) nach einem der Ansprüche 5 bis 9, wobei
die Komponente (6, 7) mindestens eines von einer Kopfplatte (6), einer Beinplatte (7), einer Sitzplatte (4) und einer Rückenplatte (5) ist.

11. Operationstisch (1), der Folgendes umfasst:
ein Operationstisch-Datenübertragungssystem (11) nach einem der Ansprüche 1 bis 4,
mindestens eine Komponente (6, 7) nach einem der Ansprüche 5 bis 10,
eine Steuerung (9) des Operationstischs (1), wobei die Steuerung (9) zum Steuern von Funktionen des Operationstischs (1) konfiguriert ist, wobei
der Datenaggregationsknoten (10) des Operationstisch-Datenübertragungssystems zum Austauschen der Daten mit der Steuerung (9) konfiguriert ist.

12. Operationstisch (1) nach Anspruch 11, wobei
der Datenaggregationsknoten (10) ein separates Gerät ist, das mit der Steuerung (9) des Operationstischs (1) verbunden ist.

## Revendications

1. Système de transmission de données de table chirurgicale (11) comprenant
une pluralité de noeuds distants (8), dans lequel chaque noeud distant (8) est rattaché à un composant (7) ou à un accessoire (12) d'une table chirurgicale (1), et
un noeud d'agrégation de données (10), dans lequel
chacun de la pluralité de noeuds distants (8) est configuré pour échanger des données avec d'autres noeuds distants (8) et avec le noeud d'agrégation de données (10) par une première communication filaire ou sans fil,
chacun de la pluralité de noeuds distants (8) est configuré pour transmettre des données d'identification du composant (7) ou de l'accessoire (12) au noeud d'agrégation de données (10), soit indirectement par l'intermédiaire d'au moins un autre noeud distant, soit directement avec le noeud d'agrégation de données (10), et
le noeud d'agrégation de données (10) est configuré pour pouvoir échanger les données avec au moins un contrôleur (9) de la table chirurgicale (1) et une passerelle de réseau hospitalier (14) par une seconde communication filaire ou sans fil.

2. Système de transmission de données de table chirurgicale (11) selon la revendication 1, dans lequel le noeud distant (8) est configuré en outre pour transmettre des données de capteur à partir du composant (7) ou de l'accessoire (12) au noeud d'agrégation de données (10), soit indirectement par l'intermédiaire d'au moins un autre noeud distant, soit directement avec le noeud d'agrégation de données (10).

3. Système de transmission de données de table chirurgicale (11) selon toute revendication précédente, dans lequel le noeud d'agrégation de données (10) et le noeud distant (8) comprennent respectivement au moins un émetteur et un récepteur pour la communication radio entre le noeud d'agrégation de données (10) et le noeud distant (8).

4. Système de transmission de données de table chirurgicale (11) selon toute revendication précédente, dans lequel le noeud d'agrégation de données (10) comprend au moins un émetteur et un récepteur pour la communication radio avec le contrôleur (9) de la table chirurgicale ou la passerelle de réseau hospitalier (14).

5. Composant (6, 7) d'une table chirurgicale (1), le composant (6, 7) comprenant un noeud distant (8) du système de transmission de données de table chirurgicale (1) selon l'une quelconque des revendications 1 à 4.

6. Composant (6, 7) selon la revendication 5, le composant (6, 7) comprenant un dispositif de mémoire (20) configuré pour stocker les données d'identification du composant (6, 7).

7. Composant (6, 7) selon la revendication 5 ou 6, le composant (6, 7) comprenant un capteur (13 - 13"") configuré pour transmettre des données de capteur au noeud distant (8).

8. Composant (6, 7) selon la revendication 7, dans lequel le capteur (13 - 13"") est au moins l'un des éléments suivants : un capteur de cartographie de pression (13), un capteur de température (13'), un capteur de poids de patient (13"), un capteur de mesure d'angle (13'") et un capteur de détection de collision (13ʺʺ).

9. Composant (6, 7) selon l'une quelconque des revendications 5 à 8,
le composant (6, 7) comprenant une batterie (18) configurée pour fournir de l'énergie au noeud distant (8).

10. Composant (6, 7) selon l'une quelconque des revendications 5 à 9,
le composant (6, 7) étant au moins l'une d'une plaque de tête (6), d'une plaque de pied (7), d'une plaque d'assise (4) et d'une plaque arrière (5).

11. Table chirurgicale (1) comprenant
un système de transmission de données de table chirurgicale (11) selon l'une quelconque des revendications 1 à 4,
au moins un composant (6, 7) selon l'une quelconque des revendications 5 à 10,
un contrôleur (9) de la table chirurgicale (1), le contrôleur (9) étant configuré pour commander des fonctions de la table chirurgicale (1),
dans lequel le noeud d'agrégation de données (10) du système de transmission de données de table chirurgicale est configuré pour échanger des données avec le contrôleur (9).

12. Table chirurgicale (1) selon la revendication 11, dans laquelle le noeud d'agrégation de données (10) est un dispositif séparé connecté au contrôleur (9) de la table chirurgicale (1).
